(19) Europäisches Patentamt
European Patent Office
Office européen des brevets

(11) EP 1 283 698 B1

(12) **FASCICULE DE BREVET EUROPEEN**

(45) Date de publication et mention de la délivrance du brevet:
**02.08.2006 Bulletin 2006/31**

(21) Numéro de dépôt: **01936573.3**

(22) Date de dépôt: **18.05.2001**

(51) Int Cl.:
***A61Q 5/06*** *(2006.01)* ***A61K 8/04*** *(2006.01)*
***A61K 8/81*** *(2006.01)*

(86) Numéro de dépôt international:
**PCT/FR2001/001525**

(87) Numéro de publication internationale:
**WO 2001/089470 (29.11.2001 Gazette 2001/48)**

(54) **UTILISATION EN COSMETIQUE DE COPOLYMERES ETHYLENIQUES SEQUENCES A CARACTERE ELASTIQUE ET COMPOSITIONS LES CONTENANT**

ZUSAMMENSETZUNG VON ETHYLENISCHEN BLOCKCOPOLYMEREN MIT ELASTISCHEN EIGENSCHAFTEN UND DEREN VERWENDUNG IN KOSMETIKA

USE IN COSMETICS OF BLOCK ETHYLENE COPOLYMERS WITH ELASTIC CHARACTER AND COMPOSITIONS CONTAINING SAME

(84) Etats contractants désignés:
**AT BE CH CY DE DK ES FI FR GB GR IE IT LI LU MC NL PT SE TR**

(30) Priorité: **23.05.2000 FR 0006534**

(43) Date de publication de la demande:
**19.02.2003 Bulletin 2003/08**

(73) Titulaire: **L'ORÉAL**
**75008 Paris (FR)**

(72) Inventeur: **MOUGIN, Nathalie**
**F-75011 Paris (FR)**

(74) Mandataire: **Casalonga, Axel**
**Bureau Casalonga & Josse**
**Bayerstrasse 71/73**
**80335 München (DE)**

(56) Documents cités:
**WO-A-00/40628**
**WO-A-00/71591**
**FR-A- 2 746 640**
**US-A- 5 711 940**

EP 1 283 698 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne l'utilisation dans le domaine cosmétique de copolymères éthyléniques séquencés à caractère élastique ainsi que des compositions cosmétiques contenant de tels copolymères.

Certains copolymères à blocs ou copolymères séquencés sont connus pour être des élastomères thermoplastiques, c'est-à-dire des polymères alliant l'élasticité d'un caoutchouc vulcanisé à une plasticité ou fusibilité à chaud (Thermoplastic Elastomers : Comprehensive Review, Legge N.R., Holden G., éditions Hense Munich, 1987).

Les propriétés élastiques de ce type de polymère découlent de l'association d'au moins une séquence dite "souple" apportant les propriétés élastiques et d'au moins une séquence dite "rigide" assurant, par auto-association, la réticulation physique réversible des chaînes macromoléculaires.

La demande WO 98/38981 divulgue des gels de solvants hydrocarbonés contenant des élastomères thermoplastiques et notamment des copolymères séquencés styrène-butadiène-styrène, styrène-isoprène-styrène et styrène-éthylène/butylène-styrène commercialisés par la Shell Chemical Company sous la dénomination Kraton® . Dans ces milieux hydrocarbonés, les copolymères jouent le rôle d'agent épaississant et gélifiant ce qui ne permet pas de les formuler à des teneurs élevées.

Ces polymères présentent en outre l'inconvénient d'être insolubles dans la plupart des solvants utilisés dans le domaine cosmétique, tels que les alcools, éthers, esters et/ou l'eau. Par ailleurs, la synthèse de ces copolymères séquencés se fait par polymérisation anionique, méthode difficile à mettre en oeuvre.

De nouvelles techniques de polymérisation radicalaire ont été mises au point récemment telles que la polymérisation contrôlée ("New Method of Polymer Synthesis", Blackie Academic & Professional, Londres, 1995, volume 2, page 1, ou *Trends Polym. Sci.* 4, page 183 (1996) de C. J. Hawker), et notamment la polymérisation radicalaire par transfert d'atome *(JACS,* 117, page 5614 (1995), de Matyjasezwski *et al.).* Ces techniques permettent à présent de synthétiser par voie radicalaire une très grande variété de copolymères séquencés "sur mesure" dans des conditions opératoires plus facilement industrialisables que cela n'était le cas pour la polymérisation anionique ou cationique, et permettent ainsi un ajustement des propriétés physico-chimique des polymères en fonction de l'application envisagée.

En incorporant ces nouveaux copolymères séquencés dans des compositions cosmétiques, la demanderesse a découvert que certains copolymères éthyléniques séquencés à caractère élastique décrits plus en détail ci-dessous, avaient des propriétés cosmétiques très intéressantes. De manière générale, ils conduisent à des systèmes non collants. Utilisés dans des laques pour cheveux, ils en améliorent à la fois le pouvoir coiffant et la souplesse. Ils augmentent la résistance aux chocs des vernis à ongles et améliorent la tenue d'une grande variété de compositions de maquillage sans provoquer chez l'utilisateur un sentiment d'inconfort.

L'invention a par conséquent pour objet l'utilisation, en cosmétique, de copolymères éthyléniques séquencés à caractère élastique comportant

(a) au moins une séquence rigide ayant une température de transition vitreuse ($T_g$) supérieure ou égale à 20 °C, constituée de motifs dérivés d'un ou de plusieurs monomères éthyléniques et

(b) au moins une séquence souple ayant une température de transition vitreuse ($T_g$) inférieure à 20 °C, constituée de motifs dérivés d'un ou de plusieurs monomères éthyléniques, lesdits copolymères permettant l'obtention d'un film ayant une recouvrance instantanée comprise entre 5 et 100 % à l'exclusion des copolymères séquencés ayant des séquences souples constituées exclusivement de motifs d'éthylène, de propylène, de butylène, de butadiène et/ou d'isoprène.

Elle a en outre pour objet des compositions cosmétiques comprenant ces copolymères éthyléniques séquencés à caractère élastique.

Un autre objet de l'invention est l'utilisation des copolymères éthyléniques séquencés à caractère élastique comportant au moins une séquence rigide ayant une température de transition vitreuse ($T_g$) supérieure ou égale à 20 °C et au moins une séquence souple ayant une température de transition vitreuse ($T_g$) inférieure à 20 °C, pour améliorer la souplesse et le pouvoir coiffant d'une laque pour cheveux, augmenter la résistance aux chocs d'un vernis à ongles, ou améliorer la tenue d'une composition de maquillage.

D'autres objets apparaîtront à la lecture de la description et des exemples qui suivent.

L'expression "motifs dérivés d'un monomère" telle qu'elle est utilisée dans la présente invention désigne les motifs constitutifs du polymère obtenus par polyméristion dudit monomère.

Les copolymères éthyléniques séquencés utilisés en cosmétique conformément à l'invention sont des copolymères comportant au moins deux séquences de monomères qui diffèrent par leur température de transition vitreuse, l'une ayant une température de transition vitreuse supérieure ou égale à la température ambiante (20 °C) et l'autre une température de transition vitreuse inférieure à la température ambiante. Le premier type de séquence est généralement qualifié de "rigide" car, à température ambiante, cette partie du polymère est à l'état vitreux, alors que le deuxième type de séquence, à l'état plastique à température ambiante, est dit "souple".

Comme indiqué ci-dessus, ces copolymères éthyléniques séquencés à caractère élastique sont de préférence obtenus par polymérisation radicalaire contrôlé décrite, entre autres, dans "New Method of Polymer Synthesis", Blackie Academic & Professional, Londres, 1995, volume 2, page 1, ou dans *Trends Polym. Sci.* 4, page 183 (1996) de C. J. Hawker.

La polymérisation radicalaire controlée permet de réduire les réactions de désactivation de l'espèce radicalaire en croissance, en particulier l'étape de terminaison, réactions qui, dans la polymérisation radicalaire classique, interrompent la croissance de la chaîne polymérique de façon irréversible et sans contrôle.

Afin de diminuer la probabilité des réactions de terminaison, il a été proposé de bloquer de façon transitoire et réversible, l'espèce radicalaire en croissance, en formant des espèces actives dites "dormantes" sous forme de liaison de faible énergie de dissociation.

Ainsi, la polymérisation peut être effectuée selon la technique de transfert d'atome, ou par réaction avec un nitroxyde, ou bien encore selon la technique de *"reversible addition-fragmentation chain transfert".*

La technique de polyméristion radicalaire par transfert d'atomes, aussi connue sous l'abréviation ATRP, consiste à bloquer l'espèce radicalaire en croissance sous forme de liasion de type C-halogénure (en présence de complexe métal/ligand). Ce type de polymérisation se traduit par un contrôle de la masse des polymères formés et par un faible indice de polydispersité.

D'une manière générale, la polymérisation radicalaire par transfert d'atomes s'effectue par polymérisation d'un ou de plusieurs monomères polymérisables par voie radicalaire, en présence

- d'un amorceur ayant au moins un atome d'halogène transférable,
- d'un composé comprenant un métal de transition susceptible de participer à une étape de réduction avec l'amorceur et une chaîne polymérique "dormante", et
- d'un ligand pouvant être choisi parmi les composés comprenant un atome d'azote (N), d'oxygène (O), de phosphore (P) ou de soufre (S), susceptibles de se coordonner par une liaison $\sigma$ audit composé comprenant un métal de transition, la formation de liaisons directes entre ledit composé comprenant un métal de transition et le polymère en formation étant évitées.

L'atome d'halogène est de préférence un atome de chlore ou de brome.

Ce procédé est en particulier décrit dans la demande WO 97/18247 et dans l'article de Matyjasezwski *et al.* publié dans *JACS,* 117, page 5614 (1995).

La technique de polymérisation radicalaire par réaction avec un nitroxyde consiste à bloquer l'espèce radicalaire en croissance sous forme de liaison de type C-O $NR_1R_2$, $R_1$ et $R_2$ pouvant être, indépendamment l'un de l'autre, un radical alkyle ayant de 2 à 30 atomes de carbone ou formant l'un et l'autre, avec l'atome d'azote, un cycle ayant de 4 à 20 atomes de carbone, comme par exemple un cycle 2,2,6,6-tétraméthylpipéridinyle. Cette technique de polymérisation est notamment décrite dans les articles "Synthesis of nitroxy-functionalized polybutadiène by anionic polymerization using a nitroxy-functionalized terminator", publié dans *Macromolecules* 1997, volume 30, pages 4238 - 4242, et "Macromolecular engineering via living free radical polymerizations" publié dans *Macromol. Chem. Phys.* 1998, vol. 199, pages 923 - 935, ou bien encore dans la demande WO-A-99/03894.

La technique de polymérisation RAFT (*reversible addition-fragmentation chain transfert)* consiste à bloquer l'espèce radicalaire en croissance sous forme de liaison de type C-S. On utilise pour cela des composés dithio comme des thiobenzoates, des dithiocarbamates ou des disulfures de xanthanes. Cette technique est notamment décrite dans la demande WO-A-98/58974 et dans l'article "A more versatile route to block copolymers and other polymers of complexe architecture by living radical polymerization : the RAFT process", publié dans *Macromolecules, 1999,* volume 32, pages 2071 - 2074.

La nature et la qualité des monomères, amorceurs, composés comprenant le métal de transition et le ou les ligands serons choisis par l'homme du métier sur la base de ses connaissances générales en fonction du résultat recherché.

Les températures de transition vitreuse des séquences rigides et souples des copolymères utilisés dans la présente invention sont mesurées par analyse enthalpique différentielle (DSC, *différential scanning calorimetry)* selon la norme ASTM D3418-97.

Pour que les copolymères séquencés défnis ci-dessus présentent les propriétés élastiques intéressantes pour une utilisation dans le domaine cosmétique, les séquences rigides et les séquences souples doivent être non miscibles, c'est-à-dire incompatibles les unes avec les autres. Cette incompatibilité thermodynamique est la condition *sine qua non* pour la formation de microdomaines de séquences rigides jouant le rôle de points de réticulation physique du réseau de polymères. Ces points de réticulation physique assurent le caractère élastique du système macromoléculaire, c'est-à-dire son retour, au moins partiel, à l'état initial après un étirement.

Le paramètre physique caractérisant les propriétés élastiques des copolymères séquencés ci-dessus est leur recouvrance en traction. Cette recouvrance est déterminée par essai de fluage en traction consistant à étirer rapidement

une éprouvette jusqu'à un taux d'allongement prédéterminé, puis à relâcher la contrainte et à mesurer la longueur de l'éprouvette.

L'essai de fluage utilisé pour la caractérisation des copolymères séquencés à caractère élastique de la présente invention se déroule de la manière suivante :

On utilise, comme éprouvette, un film du copolymère ayant une épaisseur de 500 $\pm$ 50 $\mu$m, découpé en bandes de 80 mm x 15 mm. Ce film de copolymère est obtenu par séchage, à une température de 22 $\pm$ 2 °C et à une humidité relative de 50 $\pm$ 5 %, d'une solution ou dispersion à 6 % en poids dudit copolymère dans de l'eau ou de l'éthanol.

Chaque bande est fixée entre deux mors, distants de 50 $\pm$ 1 mm l'un de l'autre, et est étirée à une vitesse de 20 mm/minute (dans les conditions de température et d'humidité relative ci-dessus) jusqu'à un allongement de 50 % ($\varepsilon_{max}$), c'est-à-dire jusqu'à 1,5 fois sa longueur initiale. On relâche alors la contrainte en imposant une vitesse de retour égale à la vitesse de traction, soit 20 mm/minute, et on mesure l'allongement de l'éprouvette (exprimé en % par rapport à la longueur initiale) immédiatement après retour à charge nulle ($\varepsilon_i$).

La recouvrance instantanée ($R_i$) est calculée à l'aide de la formule suivante :

$$R_i\ (\%) = ((\varepsilon_{max} - \varepsilon_i)/\varepsilon_{max}) \times 100$$

La valeur de la recouvrance instantanée dépend de nombreux facteurs tels que la nature, le nombre, la disposition et la proportion relative des séquences rigides et souples, ou encore la masse molaire du polymère. Les copolymères séquencés à caractère élastique de la présente invention ont généralement une recouvrance instantanée ($R_i$), mesurée dans les conditions indiquées ci-dessus, comprise entre 5 et 100 %, de préférence comprise entre 5 et 95 %, plus particulièrement entre 10 et 90 %, mieux encore entre 20 et 80 % et idéalement entre 55 et 78 %.

Selon la présente invention, chaque séquence peut être constituée d'un seul ou de plusieurs types de monomères différents, c'est-à-dire il peut s'agir d'une séquence de type homopolymère ou de type copolymère statistique ou alterné. Chaque séquence, bien que constituée éventuellement de plusieurs monomères distincts, ne présente qu'une seule température de transition vitreuse.

Dans la présente invention l'écart entre les températures de transition vitreuse de ces deux types de séquences, à savoir des séquences rigides et des séquences souples, est de préférence au moins égal à 20 °C, notamment compris entre 20 et 160 °C, en particulier supérieur ou égal à 50 °C, notamment compris entre 50 °C et 160 °C, et idéalement supérieur ou égal à 100 °C, notamment compris entre 100 et 160 °C.

Les copolymères éthyléniques séquencés à caractère élastique de la présente invention peuvent être choisis parmi

- les copolymères biséquencés de formule AB,
- les copolymères triséquencés de formule ABA ou BAB et
- les copolymères polyséquencés de formule $(AB)_n$, $B(AB)_n$ ou $(AB)_nA$,

où A représente une séquence rigide telle que définie ci-dessus, B représente une séquence souple telle que définie ci-dessus et n est au moins égal à deux, de préférence égal à 2 ou 3, les séquences A d'un même polymère pouvant être identiques ou différentes et les séquences B d'un même polymère pouvant être identiques ou différentes.

Dans la présente invention, on préfère tout particulièrement l'utilisation de copolymères triséquencés de structure ABA, c'est-à-dire de copolymères constitués de deux séquences rigides (A), identiques ou différentes, ayant chacune une température de transition vitreuse supérieure ou égale à 20°C, encadrant une séquence centrale souple (B) ayant une température de transition vitreuse inférieure à 20 °C.

Les séquences A (rigides) représentent de préférence de 10 à 60 % en poids et en particulier de 15 à 50 % en poids du copolymère séquencé final et les séquences B (souples) représentent par conséquent de préférence de 40 à 90 % en poids et en particulier de 50 à 85 % en poids du copolymère séquencé final.

Les copolymères éthyléniques séquencés à caractère élastique utilisés en cosmétique conformément à la présente invention comportent au moins une séquence rigide ayant une température de transition vitreuse ($T_g$) supérieure ou égale à 20 °C, et au moins une séquence souple ayant une température de transition vitreuse ($T_g$) inférieure à 20 °C, constituées de motifs dérivés d'un ou de plusieurs monomères éthyléniques choisis parmi ceux de formule

(I) $R^1R^2C{=}CR^3R^4$

dans laquelle

$R^1$, $R^2$, $R^3$ et $R^4$ représentent indépendamment l'un de l'autre chacun

- un atome d'hydrogène ou d'halogène,
- un groupe alkyle en $C_{1-20}$, pouvant être substitués par 1 ou plusieurs atomes d'halogène ou un ou plusieurs groupes OH,
- un groupe alcényle ou alcynyle $\alpha,\beta$-insaturé, linéaire ou ramifié, comportant de 2 à 10 atomes de carbone et pouvant être substitués par un ou plusieurs atomes d'halogène,
- un groupe cycloalkyle en $C_{3-8}$ pouvant être substitué par un ou plusieurs atomes d'halogène,
- un groupe cyano,
- un groupe aryle,
- un groupe hétérocyclique 4 à 12 chaînons contenant un ou plusieurs atomes de N, O, S et P,
- un groupe $-C(=Y)R^5$, $-CH_2C(=Y)R^5$, $-C(=Y)NR^6R^7$, $-YC(=Y)R^5$, $-NR^6C(=Y)R^5$, $-SOR^5$, $-SO_2R^5$, $-OSO_2R^5$, $-NR^8SO_2R^5$, $-PR^5_2$, $-P(=Y)R^5_2$, $-YPR^5_2$, $-YP(=Y)R^5_2$ ou $-NR^8_2$ éventuellement quatemisé par un $R^8$ supplémentaire où

  Y représente un groupe $NR^8$, S ou O,

  $R^5$ représente un groupe alkyle, alcoxy ou alkylthio en $C_{1-20}$ éventuellement hydroxylé, mono- ou poly (alkylèneoxy) éventuellement éthérifié, hydroxyle, -OM (avec M = métal alcalin), aryloxy ou hétérocyclyloxy,

  $R^6$ et $R^7$ représentent indépendamment l'un de l'autre un atome d'hydrogène ou un groupe alkyle en $C_{1-20}$ ou forment avec l'atome d'azote auquel ils sont liés un cycle à 3 à 8 chaînons, et

  $R^8$ représente un atome d'hydrogène, un groupe alkyle en $C_{1-20}$, ou aryle,

- un groupe $-C(=O)-X-R^9-Z$ ou $-R^9-Z$ où

  $R^9$ représente un radical divalent hydrocarboné en $C_{1-20}$ saturé ou insaturé, linéaire, ramifié ou cyclique, éventuellement halogéné et pouvant comporter un ou plusieurs hétéroatomes,

  X représente un groupe $NR^{10}$ ou un atome d'oxygène,

  Z représente un groupe $-N(R^{10})_2$, $-S-R^{10}$ ou $P(R^{10})_2$ où chaque $R^{10}$ représente indépendamment un groupe hydrocarboné en $C_{1-20}$ saturé ou insaturé, linéaire, ramifié ou cyclique, éventuellement halogéné et pouvant comporter un ou plusieurs hétéroatomes, l'atome d'azote de X et Z pouvant être protoné ou quaternisé par des radicaux alkyle en $C_{1-20}$,

- un groupe $-R^9-NR^{10}$-Acide ou $-C(=O)-X-R^9-NR^{10}$-Acide où Acide représente une fonction acide carboxylique, sulfonique ou phosphonique et $R^9$ et $R^{10}$ ont chacun la signification indiquée ci-dessus,
- un radical comprenant au moins un atome de silicium et notamment des radicaux -R-siloxane, -CONHR-siloxane, -COOR-siloxane, ou
  -OCO-R-siloxane, où R est un radical alkyle, alkylthio ou alcoxy en $C_{1-20}$, aryloxy ou hétérocyclyloxy.

Sont toutefois exclus de la présente invention des copolymères séquencés ayant des séquences souples constituées exclusivement de motifs d'éthylène, de propylène, de butylène, de butadiène et/ou d'isoprène.

De préférence, la ou les séquences rigides des copolymères éthyléniques séquencés à caractère élastique de la présente invention sont constituées de motifs d'un ou de plusieurs monomères éthyléniques choisis parmi

- l'acide acrylique ou méthacrylique,
- les méthacrylates d'alkyle en $C_{1-20}$ à chaîne linéaire, ramifiée

ou cyclique, tels que le méthacrylate de méthyle, le méthacrylate d'éthyle, le méthacrylate de propyle, le méthacrylate de butyle, le méthacrylate d'isobutyle, le méthacrylate de *tert*-butyle et le méthacrylate de cyclohexyle,

- les méthacrylates d'hydroxyalkyle en $C_{1-4}$, tel que le (méth)acrylate de 2-hydroxyéthyle et le méthacrylate de 2-hydroxypropyle,
- certains esters de vinyle tels que l'acétate de vinyle, le propionate de vinyle, le benzoate de vinyle et le *tert*-butylbenzoate de vinyle,
- les monomères hétérocycliques, tels que la N-vinylpyrrolidone, le vinylcaprolactame, les vinyl-N-(alkyle en $C_{1-6}$)-pyr-

roles, les vinyloxazoles, les vinylthiazoles, les vinylpyrimictïnes, les vinylimidazoles,

- le (méth)acrylamide,
- certains méthacrylamides aliphatiques, cycloaliphatiques ou aromatiques, tels que le *tert*-butylacrylamide et les di (alkyle en $C_{1-4}$)-méthacrylamides,
- le styrène,
- certains styrènes substitués,
- les monomères (méth)acryliques ou vinyliques à groupe fluoré

ou perfluoré tels que le méthacrylate de perfluorooctyléthyle, ou les (méth)acrylamides à groupe fluoré ou perfluoré,

- les monomères (méth)acryliques ou vinyliques siliconés tels que le méthacryloxypropyltris(triméthylsiloxy)silane, ou les (méth)acrylamides siliconés,
- les monomères acryliques ou vinyliques comportant une fonction amine éventuellement neutralisée ou quaternisée, tels que le (méth)acrylate de diméthylaminoéthyle, le diméthylaminoéthylméthacrylamide, la vinylamine, la vinylpyridine, le chlorure de diallyldiméthylammonium,
- les carboxybétaïnes ou sulfobétaïnes éthyléniques obtenues par exemple par quaternisation de monomères à insaturation éthylénique comportant une fonction amine par des sels de sodium d'acide carboxylique à halogène mobile (p. ex. chloroacétate de sodium) ou par des sulfones cycliques (p. ex. propanesultone).

On peut citer à titre d'exemples de séquences rigides préférées, les séquences poly(méthacrylate de méthyle), polystyrène et poly(méthacrylate de perfluorooctyléthyle).

De préférence, la ou les séquences souples des copolymères éthyléniques séquencés à caractère élastique de la présente invention sont constituées de motifs dérivés d'un ou de plusieurs monomères éthyléniques choisis parmi

- les acrylates d'alkyle en $C_{1-20}$ à chaîne linéaire, ramifiée ou cyclique, tels que l'acrylate de méthyle, l'acrylate d'éthyle, l'acrylate de propyle, l'acrylate de butyle, acrylate de 2-éthylhexyle, l'acrylate d'isobutyle et l'acrylate de *tert*-butyle,
- les acrylates d'aryle en $C_{6-20}$,
- les acrylates d'hydroxyalkyle en $C_{1-4}$, tels que l'acrylate de 2-hydroxyéthyle et l'acrylate de 2-hydroxypropyle,
- les (méth)acrylates de mono-, di- ou poly(éthylèneglycol) à extrémité hydroxyle éventuellement éthérifiée, tels que les (méth)acrylates d'éthylèneglycol, de diéthylèneglycol ou de polyéthylèneglycol,
- certains (méth)acrylamides aliphatiques, cycloaliphatiques ou aromatiques, tels que l'undécylacrylamide ou le N-octylacrylamide,
- certains éthers de vinyle tels que le vinylisobutyléther,
- certains styrènes substitués,
- les monomères acryliques ou vinyliques à groupe fluoré ou perfluoré, tels que les esters acryliques à chaîne perfluoroalkyle comme l'acrylate de perfluorooctyléthyle,
- les monomères acryliques ou vinyliques siliconés, tels que l'acryloxypropylpolydiméthylsiloxane.

On peut citer à titre d'exemples de séquences souples préférées les séquences poly(acrylate de butyle) et poly(acrylate de 2-éthylhexyle).

Des polymères particulièrement intéressants pour les applications cosmétiques de la présente invention sont :

- les copolymères triséquencés poly(méthacrylate de méthyle-b-acrylate de butyle-b-méthacrylate de méthyle)
- les copolymères triséquencés poly(méthacrylate de méthyle-b-acrylate d'isobutyle-b-méthacrylate de méthyle) et
- les polymères triséquencés poly(méthacrylate de méthyle-b-acrylate de butyle-b-styrène).
  L'invention a également pour objet des compositions cosmétiques contenant les copolymères éthyléniques séquencés à caractère élastique décrits ci-dessus.
  Ces compositions cosmétiques contiennent les copolymères éthyléniques séquencés élastiques sous forme dissoute ou dispersée dans un milieu solvant approprié, physiologiquement acceptable.
  On peut citer à titre d'exemple de tels solvants l'eau, les cétones telles que la méthyléthylcétone, la méthylisobutylcétone, la diisobutylcétone, l'isophorone, la cyclohexanone ou l'acétone, les alcools inférieurs tels que l'éthanol, l'isopropanol, le diacétone-alcool, le 2-butoxyéthanol

ou le cyclohexanol, les alkylèneglycols tels que l'éthylèneglycol, le propylèneglycol ou le pentylèneglycol, les éthers d'alkylèneglycol tels que l'éther monométhylique de propylènglycol, l'acétate de l'éther monométhylique de propylèneglycol ou l'éther monobutylique de dipropylèneglycol, les acétates d'alkyle en $C_{2-7}$ tels que l'acétate de méthyle, l'acétate d'éthyle, l'acétate de propyle, l'acétate de butyle ou l'acétate d'isopentyle, les éthers tels que l'éther diéthylique, l'éther diméthylique ou le dichlorodiéthyléther, les alcanes tels que le décane, l'heptane, le dodécane ou le cyclohexane, les

hydrocarbures aromatiques tels que le toluène et le xylène, et les huiles volatiles telles que les huiles siliconées volatiles cycliques ou linéaires, les huiles volatiles hydrocarbonées telles que les isoparaffines, ou encore les huiles fluorées.

Les copolymères éthyléniques séquencés élastiques sont présents dans les compositions cosmétiques en des concentrations qui dépendent de leur structure chimique mais surtout du type de composition cosmétique. De manière générale, cette concentration en copolymères séquencés à caractère élastique est comprise entre 1 et 99 % en poids, de préférence entre 5 et 50 % en poids, et encore mieux entre 7 et 40 % en poids.

Les compositions cosmétiques de la présente invention peuvent en outre comprendre une phase grasse composée d'huiles, de gommes et/ou de cires.

Les huiles, corps gras liquides à température ambiante (25 °C), cosmétiquement acceptables peuvent être des huiles hydrocarbonées et/ou siliconées et/ou fluorées. Elles peuvent être d'origine animale, végétale, minérale ou synthétique.

On peut citer en particulier, seule ou en mélange :

- les huiles hydrocarbonées d'origine animale telles que le perhydrosqualène,
- les huiles hydrocarbonées végétales telles que les huiles de tournesol, de maïs, de soja, de courge, de pépins de raison, d'arachide, d'amande douce, de calophyllum, de palme, de sésame, de noisette, d'abricot, de macadamia, de ricin, d'avocat, de jojoba et de beurre de karité, les triglycérides liquides d'acides gras en $C_{4-10}$, comme les triglycérides des acides heptanoïque ou octanoïque, les triglycérides des acides caprylique/caprique comme ceux vendus par la société Stearineries Dubois ou ceux vendus sous les dénominations Miglyol® 810, 812 et 818 par la société Dynamit Nobel,
- les hydrocarbures linéaires ou ramifiés d'origine minérale ou synthétique tels que les huiles de paraffine et leurs dérivés, la vaseline, les polydécènes, l'huile de Purcellin, le polyisobutène hydrogéné tel que le parléam,
- les esters de synthèse, notamment

  - les esters d'acides gras comme les huiles de formule $R^3COOR^4$ dans laquelle $R^3$ représente le reste d'un acide gras supérieur comportant de 7 à 29 atomes de carbone et $R^4$ représente une chaîne hydrocarbonée contenant de 3 à 30 atomes de carbone, comme par exemple le myristate d'isopropyle, le palmitate de 2-éthylhexyle, le stéarate de 2-octyldodécyle, l'éruçate de 2-octyldodécyle et fisostéarate d'isostéaryle,
  - les esters hydroxylés tels que le lactate d'isostéaryle, l'hydroxystéarate d'octyle, fhydroxystéarate d'octyldodécyle, le malate de diisostéaryle et le citrate de triisocétyle,
  - les esters de polyols comme le dioctanoate de propylèneglycol, le diheptanoate de néopentylglycol, le diisononanoate de diéthylèneglycol et les esters du pentaérythritol,

- les alcools gras ayant de 12 à 26 atomes de carbone comme l'octyldodécanol, le 2-butyloctanol, le 2-héxyldécanol, le 2-undécylpentadécanol et l'alcool oléylique,
- les huiles hydrocarbonées partiellement fluorées et/ou siliconées
- les huiles siliconées telles que les polydiméthylsiloxanes, volatiles ou non, linéaires ou cycliques, les alkyldiméthicones, les silicones modifiées par des groupements aliphatiques et/ou aromatiques éventuellement fluorés, ou par des groupements fonctionnels tels que des groupements hydroxyles, thiols et/ou amine, les huiles siliconées phénylées telles que les polyphénylméthylsiloxanes ou les phényltriméthicones.

  Les huiles employées peuvent être volatiles et/ou non volatiles. Par huile volatile, on entend une huile susceptible de s'évaporer à température ambiante d'un support sur lequel elle a été appliquée, autrement dit une huile ayant une tension de vapeur mesurable à 25 °C et à 1 atmosphère, supérieure à 0 Pa, en particulier allant de 0,13 à 40 000 Pa. On peut citer notamment les huiles siliconées volatiles telles que les silicones volatiles cycliques ou linéaires, et les cyclocopolymères. On peut également citer les huiles volatiles hydrocarbonées telles que les isoparaffines, et les huiles fluorées volatiles.

  Parmi les gommes et/ou cires cosmétiquement acceptables susceptibles d'être utilisées on peut citer
- les gommes de silicone,
- les cires d'origine animale, végétale, minérale ou synthétique telles que les cires microcristallines, la paraffine, le pétrolatum, la vaseline, l'ozokérite, la cire de lignite, la cire d'abeille, la lanoline et ses dérivés, la cire de Candellila, la cire d'Ouricury, la cire de Carnauba, la cire du Japon, le beurre de cacao, la cire de fibres de liège, la cire de canne à sucre, les huiles hydrogénées concrètes à 25 °C, les esters gras et glycérides concrets à température ambiante, les cires de polyéthylène et les cires obtenues par synthèse de Fischer-Tropsch, les lanolines,
- les cires de silicone et
- les cires fluorées.

Les compositions cosmétiques de la présente invention peuvent contenir en outre un ou plusieurs agents épaississants, un ou plusieurs polymères filmogènes et/ou un ou plusieurs agents plastifiants.

Une phase particulaire constituée de pigments et/ou de nacres et/ou de charges peut également être présente dans les compositions cosmétiques de la présente invention.

Par pigments, il faut comprendre des particules blanches ou colorées, minérales ou organiques destinées à colorer ou à opacifier la composition. On peut citer par exemple les dioxydes de titane, de zirconium ou de cérium, les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que les poudres d'argent ou d'aluminium. On peut également citer certaines laques tels que les sels de calcium, de baryum, d'aluminium ou de zirconium. Ces pigments sont généralement présents à raison de 0 à 15 % en poids et de préférence à raison de 8 à 10 % de la composition finale.

On entend par "charges" dans la présente invention des particules incolores ou blanches, minérales ou synthétiques, lamellaires ou non, destinées à donner du corps ou de la rigidité à la composition et/ou de conférer au maquillage de la douceur, de la matité et de l'uniformité. Les charges utilisables dans les compositions cosmétiques de la présente invention sont choisies par exemple parmi le talc, le mica, la silice, le kaolin, les poudres de Nylon et de polyéthylène, le Teflon® , l'amidon, le nitrure de bore, les microsphères de polymères telles que Expancel® de la société Nobel Industrie ou Polytrap® de la société Dow Coming, les microbilles de résine de silicone telles que Tospearls® dé la société Toshiba, le carbonate de calcium précipité, le carbonate ou hydrocarbonate de magnésium, les savons métalliques dérivés d'acides carboxyliques en $C_{8-22}$.

Les charges sont généralement utilisées à raison de 0 à 80 % en poids, de préférence à raison de 5 à 15 % en poids rapporté au poids final de la composition cosmétique.

Par nacres il, il faut comprendre des particules irisées qui réfléchissent la lumière. On peut citer par exemple la nacre naturelle, le mica recouvert d'oxyde de titane, d'oxyde de fer, de pigments naturels ou d'oxychlorure de bismuth ainsi que le mica titane coloré.

Les nacres sont généralement présentes à raison de 0 à 20 % en poids, de préférence à raison de 8 à 15 % en poids de la composition cosmétique finale.

La composition peut comprendre un certain nombre d'additifs usuellement utilisés dans le domaine cosmétique tels que des agents antioxydants, des parfums, des huiles essentielles, des conservateurs, des actifs cosmétiques lipophile ou hydrophiles, des agents hydratants, des vitamines, des colorants, des acides gras essentiels, des sphingolipides, des agents autobronzants, des filtres solaires, des agents anti-mousse, des agents séquestrants, des agents antioxydants ou des agents anti-radicalaires.

Bien entendu l'homme de métier veillera à choisir les éventuels composés complémentaires de manière à ce que les propriétés avantageuses de la composition selon l'invention ne soient pas, ou pratiquement pas altérées par l'adjonction envisagée.

Les compositions cosmétiques de la présente invention contenant les copolymères séquencés élastiques décrits ci-dessus peuvent se présenter sous n'importe quelle forme habituellement rencontrée dans le domaine cosmétique, c'est-à-dire sous forme d'une lotion, d'une suspension, d'une dispersion, d'une solution organique, aqueuse ou hydroalcoolique éventuellement épaissie ou gélifiée, d'une mousse, d'un spray, d'une émulsion huile-dans-eau, eau-dans-huile ou multiple, d'une poudre libre, compacte ou coulée, d'un solide ou d'une pâte anhydre.

Il peut s'agir plus particulièrement d'un produit de soin, d'hygiène et/ou de maquillage. Des modes de réalisation préférés des compositions cosmétiques de la présente invention sont représentés par les compositions capillaires, notamment les compositions coiffantes telles que laques, gels ou shampooings coiffants, les vernis à ongles et les compositions de maquillage du visage, du corps ou des phanères (ongles, cils, sourcils, cheveux), telles que fard à paupières ou à joues, eye-liner, mascara, poudre libre ou compacte, fond de teint, crème teintée, rouge à lèvres, stick anti-cernes etc.

Les exemples de réalisation suivants sont donnés pour illustrer la présentent invention mais n'ont aucun caractère limitatif de l'invention.

**Exemple 1**

<u>Préparation d'un amorceur de polymérisation difonctionnel</u>

On prépare un amorceur difonctionnel selon le schéma réactionnel suivant :

$$HO-(CH_2)_4-OH + 2\ C(CH_3)_2(Br)-C(=O)Br \xrightarrow{\text{THF/triéthylamine}}$$

$$(CH_3)_2BrC-C(=O)-O-(CH_2)_4-O-C(=O)-C(CH_3)_2Br$$

Pour cela, on mélange 18 g (0,2 mole) de 1,4-butanediol avec 100 g de tétrahydrofurane et on laisse le mélange s'équilibrer pendant 10 minutes à température ambiante. On ajoute ensuite lentement, sur une durée de 30 minutes, 40,4 g (0,4 moles) de triéthylamine de manière à ce que la température de la solution n'augmente pas brusquement. On ajoute ensuite très lentement, sur une durée de 3 heures et en refroidissant à 5 °C, 92 g (0,4 mole) de bromure de 2-bromoisobutyryle. Lors de cette addition, on observe un jaunissement progressif de la solution réactionnelle. On maintient l'agitation pendant la nuit à 25 °C, puis on laisse la température remonter progressivement jusqu'à la température ambiante.

On concentre la solution réactionnelle par évaporation du THF et on précipite le résidu dans de l'eau. On extrait ensuite 3 fois la phase aqueuse avec de l'éther éthylique, puis on sèche la phase éthérée sur du sulfate de magnésium.

Après évaporation de l'éther, on obtient ainsi 63 g de bis(1,4-bromoisobutyrate de n-butyle), ce qui correspond à un rendement de 80 %.

**Exemple 2**

Préparation d'un copolymère triséquencé poly(méthacrylate de méthyle-b-acrylate de butyle-b-méthacrylate de méthyle)

Etape. I : polymérisation d'acrylate de butyle

On mélange dans un réacteur hermétique, à l'abri d'oxygène, et comportant une arrivée d'azote, 0,078 g ($2.10^{-4}$ mole) d'amorceur difonctionnel préparé dans l'Exemple 1, $2,9.10^{-4}$ mole de CuBr, $5,7.10^{-4}$ mole de 2,2'-bipyridine et 30 g d'acrylate de butyle. On chauffe sous atmosphère d'azote à une température de 120 °C, on coupe l'arrivée d'azote et on maintient cette température pendant 5 heures.

Etape II : polymérisation de méthacrylate de méthyle

On ajoute ensuite au mélange réactionnel 12 g de méthacrylate de méthyle, on fait réagir pendant 3 heures à 120 °C, puis on laisse refroidir le mélange à température ambiante. On obtient 42 g d'une solution visqueuse de couleur verte que l'on dissout dans environ 100 ml de dichlorométhane. On fait passer cette solution du polymère sur un lit d'alumine neutre, puis on fait précipiter la solution limpide dans 5 volumes d'un mélange méthanol/eau (80/20).

On obtient ainsi 37 g de polymère se présentant sous une forme de pâte, ce qui correspond à un rendement de 90 % en poids.

On lave la pâte à l'heptane à chaud pour en éliminer les monomères résiduels éventuellement présents.

On détermine la masse molaire moyenne en poids et en nombre par chromatographie liquide par perméation de gel (solvant THF, courbe d'étalonnage établie avec des étalons de polystyrène linéaire). La masse molaire moyenne en nombre ($M_n$) est égale à 51 900 et la masse molaire moyenne en poids ($M_p$) est égale à 114 500.

Le copolymère présente deux températures de transition vitreuse $T_g$, la première égale à -47 °C imputable à la séquence polyacrylate de butyle, et la seconde égale à 70 °C imputable aux séquences de poly(méthacrylate de méthyle).

La recouvrance instantanée du copolymère est de 75 %.

**Exemple 3**

Préparation d'une laque

On prépare un aérosol avec 100 g d'une solution à 9 % en poids du polymère préparé dans l'Exemple 2 dans de l'éthanol et 75 g de diméthyl-éther jouant le rôle de gaz propulseur.

On pulvérise la composition sur des mèches de cheveux châtains de longueur de 18 cm et on évalue la tenue de la coiffure et l'aspect souple des mèches sur un panel de 5 personnes en utilisant une échelle de notation allant de

0 (mauvais) à 5 (excellent). Les notes obtenues sont 4 pour la tenue de la coiffure et 4 pour l'aspect souple des mèches.

**Exemple 4**

Préparation d'un vernis à ongles

On dissout le polymère obtenu dans l'Exemple 2 à raison de 25 % en poids dans de l'acétate d'éthyle.

On applique la solution de manière habituelle sur l'ongle. Le vernis séché présente une bonne résistance au vieillissement. Il ne s'use pas et reste brillant. Il s'élimine facilement à l'aide des dissolvants classiques à base d'acétone.

## Revendications

1. Utilisation, en cosmétique, de copolymères éthyléniques séquencés à caractère élastique comportant

    (a) au moins une séquence rigide ayant une température de transition vitreuse ($T_g$) supérieure ou égale à 20 °C, constituée de motifs dérivés d'un ou de plusieurs monomères éthyléniques et
    (b) au moins une séquence souple ayant une température de transition vitreuse ($T_g$) inférieure à 20 °C, constituée de motifs dérivés d'un ou de plusieurs monomères éthyléniques,

    lesdits copolylmères permettant l'obtention d'un film ayant une recouvrance instantanée comprise entre 5 et 100% à l'exclusion des copolymères séquencés ayant des séquences souples constituées exclusivement de motifs d'éthylène, de propylène, de butylène, de butadiène et/ou d'isoprène, choisis parmi les copolymères diséquencés de formule AB, les copolymères triséquencés de formule ABA ou BAB et les copolymères polyséquencés de formule $(AB)_n$, $B(AB)_n$ ou $(AB)_nA$, où chaque A représente une séquence rigide ayant une température de transition vitreuse supérieure ou égale à la température ambiante (20 °C), chaque B représente une séquence souple ayant une température de transition vitreuse inférieure à la température ambiante (20 °C) et n est au moins égal à deux, de préférence égal à 2 ou 3, les séquences A d'un même polymère pouvant être identiques ou différentes et les séquences B d'un même polymère pouvant être identiques ou différentes,

2. Utilisation selon la revendication 1, **caractérisée par le fait que** les copolymères éthyléniques séquencés à caractère élastique sont des polymères obtenus par polymérisation radicalaire contrôlée.

3. Utilisation selon la revendication 1 ou 2, **caractérisée par le fait que** ladite séquence rigide, ayant une température de transition vitreuse ($T_g$) supérieure ou égale à 20 °C, est constituée de motifs dérivés d'un ou de plusieurs monomères éthyléniques choisis parmi l'acide acrylique ou méthacrylique, les méthacrylates d'alkyle en $C_{1-20}$ à chaîne linéaire, ramifiée ou cyclique, les méthacrylates d'hydroxyalkyle en $C_{1-4}$, certains esters de vinyle, les monomères hétérocycliques, le (méth)acrylamide, certains méthacrylamides aliphatiques, cycloaliphatiques ou aromatiques, le styrène, certains styrènes substitués, les monomères (méth)acryliques ou vinyliques à groupe fluoré ou perfluoré ou les (méth)acrylamides à groupe fluoré on perfuoré, les monomères (méth)acryliques ou vinyliques siliconés ou les (méth)acrylamides siliconés, les monomères acryliques on vinyliques comportant une fonction amine éventuellement neutralisée ou quaternisée, et les carboxybetaïnes ou sulfobétaïnes ethyléniques.

4. Utilisation selon l'une des revendications précédentes, **caractérisée par le fait que** ladite séquence souple ayant une température de transition vitreuse ($T_g$) inférieure à 20°C, est constituée de motifs dérivés d'un ou de plusieurs monomères éthyléniques choisis parmi les acrylates d'alkyle en $C_{1-20}$ à chaîne linéaire, ramifiée ou cyclique, les acrylates d'aryle en $C_{6-20}$, les acrylates d'hydroxyalkyle en $C_{1-4}$, les (méth)acrylates de mono-, di- ou poly(étllylèneglycol) à extrémité hydroxyle éventuellement éthérifiée, certains (méth)acrylamides aliphatiques, cycloaliphatiques ou aromatiques, certains éthers de vinyle, certains styrènes substitués, les monomères acryliques: ou vinyliques à groupe fluoré ou perfluoré, et les monomères acryliques ou vinyliques siliconés.

5. Utilisation selon l'une quelconque des revendications précédentes, **caractérisée par le fait que** lesdits copolymères éthyléniques sont des copolymères triséquencés de formule ABA où chaque A représente indépendamment une séquence rigide ayant une température de transition vitreuse supérieure ou égale à la température ambiante (20 °C), et B représente une séquence souple ayant une température de transition vitreuse inférieure à la température ambiante (20 °C).

6. Utilisation selon l'une quelconque des revendications précédentes, **caractérisée par le fait que** les copolymères

éthyléniques séquencés sont choisis parmi les

- les copolymères triséquencés poly(méthacrylate de méthyle-b-acrylate de butyle-b-méthacrylate de méthyle)
- les copolymères triséquencés poly(méthacrylate de méthyle-b-acrylate d'isobutyle-b-méthacrylate de méthyle) et
- les polymères triséquencés poly(méthacrylate de méthyle-b-acrylate de butyle-b-styrène).

7. Utilisation selon l'une quelconque des revendications précédentes, **caractérisée par le fait que** les séquences rigides A sont incompatibles, c'est-à-dire non miscibles, avec les séquences souples B.

8. Utilisation selon l'une quelconque des revendications précédentes, **caractérisée par le fait que** l'écart entre les températures de transition vitreuse des séquences rigides et des séquences souples est au moins égal à 20 °C, de préférence supérieur à 50 °C et idéalement supérieur à 100 °C.

9. Utilisation selon l'une quelconque des revendications précédentes, **caractérisée par le fait que** lesdits polymères séquencés présentent une recouvrance instantanée comprise entre 5 et 95 %, de préférence entre 10 et 90 %, en particulier entre 20 et 80 % et idéalement entre 55 et 78 %.

10. Utilisation selon l'une quelconque des revendications précédentes, **caractérisée par le fait que** les séquences A représentent de 10 à 60 % en poids et en particulier de 15 à 50 % en poids du copolymère séquencé final et les séquences B représentent de 40 à 90 % en poids et en particulier de 50 à 85 % en poids du copolymère séquence final.

11. Composition cosmétique comprenant, dans un milieu physiologiquement acceptable, au moins un copolymère éthylénique séquencé à caractère élastique comportant

(a) au moins une séquence rigide ayant une température de transition vitreuse ($T_g$) supérieure ou égale à 20 °C, constituée de motifs dérivés d'un ou de plusieurs monomères éthyléniques et
(b) au moins une séquence souple ayant une température de transition vitreuse ($T_g$) inférieure à 20°C, constituée de motifs dérivés d'un ou de plusieurs monomères éthyléniques,

lesdits copolymères permettant l'obtention d'un film ayant une recouvrance instantanée comprise entre 5 et 100 % à l'exclusion des copolymères séquencés ayant des séquences souples constituées exclusivement de motifs d'éthylène, de propylène, de butylène, de butadiène et/ou d'isoprène, choisis parmi les copolymères diséquencés de formule AB, les copolymères triséquencés de formule ABA ou BAB et les copolymères polyséquencés de formule $(AB)_n$, où chaque A représente une séquence rigide ayant une température de transition vitreuse supérieure ou égale à la température ambiante (20 °C), chaque B représente une séquence souple ayant une température de transition vitreuse inférieure à la température ambiante (20 °C) et n est au moins égal à deux, de préférence égal à 2 ou 3, les séquences A d'un même polymère pouvant être identiques ou différentes et les séquences B d'un même polymère pouvant être identiques ou différentes

12. Composition cosmétique selon la revendication 11, **caractérisée par le fait que** les copolymères éthyléniques séquencés à caractère élastique sont des polymères obtenus par polymérisation radicalaire contrôlée.

13. Composition selon la revendication 11 ou 12, **caractérisée par le fait que** ladite séquence rigide ayant une température de transition vitreuse ($T_g$) supérieure ou égale à 20 °C, est constituée de motifs dérivés d'un ou de plusieurs monomères éthyléniques choisis parmi l'acide acrylique ou méthacrylique, les méthacrylates d'alkyle en $C_{1-20}$ à chaîne linéaire, ramifiée ou cyclique, les méthacrylates d'hydroxyalkyle en $C_{1-4}$, certains esters de vinyle, les monomères hétérocycliques, le (méth)acrylamide, certains méthacrylamides aliphatiques, cycloaliphatiques ou aromatiques, le styrène, certains styrènes substitués, les monomères (meth)acryliques ou vinyliques à groupe fluoré ou perfluoré ou les (méth)acrylamides à groupe fluoré ou perfluoré, les monomères (méth)acryliques ou vinyliques siliconés ou les (méth)acrylamides siliconés, les monomères acryliques ou vinyliques comportant une fonction amine éventuellement neutralisée ou quaternisée, et les carboxybétaïnes ou sulfobétaïnes éthyléniques.

14. Composition cosmétique selon l'une des revendications 11 à 13, **caractérisée par le fait que** ladite séquence souple ayant une température de transition vitreuse ($T_g$) inférieure à 20 °C, est constituée de motifs dérivés d'un ou de plusieurs monomères éthyléniques choisis parmi les acrylates d'alkyle en $C_{1-20}$ à chaîne linéaire, ramifiée ou cyclique, les acrylates d'aryle en $C_{6-20}$, les acrylates d'hydroxyalkyle en $C_{1-4}$, les (méth)acrylates de mono-, di- ou poly(éthylèneglycol) à extrémité hydroxyle éventuellement éthérifiée, certains (méth)acrylamides aliphatiques,

cycloaliphatiques ou aromatiques, certains éthers de vinyle, certains styrènes substitués, les monomères acryliques ou vinyliques à groupe fluoré ou perfluoré, et les monomères acryliques ou vinyliques siliconés.

**15.** Compositions selon l'une quelconque des revendications 11 à 14, **caractérisée par le fait que** les copolymères éthyléniques sont des copolymères triséquencés de formule ABA où chaque A représente indépendamment une séquence rigide ayant une température de transition vitreuse supérieure ou égale à la température ambiante (20 °C), et B représente une séquence souple ayant une température de transition vitreuse inférieure à la température ambiante (20 °C).

**16.** Composition selon l'une quelconque des revendications 11 à 15, **caractérisée par le fait que** les séquences rigides A sont incompatibles c'est-à-dire non miscibles, avec les séquences souples B.

**17.** Composition cosmétique selon l'une quelconque des revendications 11 à 16, **caractérisée par le fait que** les copolymères éthyléniques sont choisis parmi les

- les copolymères triséquencés poly(méthacrylate de méthyle-b-acrylate de butyle-b-méthacrylate de méthyle)
- les copolymères triséquencés poly(méthacrylate de methyle-b-acrylate d'isobutyle-b-méthacrylate de méthyle) et
- Les polymères triséquencés poly(méthacrylate de méthyle-b-acrylate de butyle-b-styrène).

**18.** Composition selon l'une quelconque des revendications 1 à 17, **caracterisée par le fait que** l'écart entre les températures de transition vitreuse des séquences rigides et des séquences souples est au moins égal à 20 °C, de préférence supérieur à 50 °C et idéalement supérieur à 100 °C.

**19.** Composition selon l'une quelconque des revendications 11 à 18, **caractérisée par le fait que** lesdits polymères séquences à caractère élastique présentent une recouvrance instantanée comprise entre 5 et 95 %, de préférence entre 10 et 90 %, en particulier entre 20 et 80 % et idéalement entre 55 et 78 %.

**20.** Composition selon l'une quelconque des revendications 11 à 19, **caractérisée par le fait que** les séquences A représentent de 10 à 60 % en poids et en particulier de 15 à 50 % en poids du copolymère séquencé final et les séquences B représentent de 40 à 90 % en poids et en particulier de 50 à 85 % en poids du copolymère séquencé final.

**21.** Composition cosmétique selon l'une quelconque des revendications 11 à 20, **caractérisée par le fait qu'**elle contient de 1 à 99 % en poids, de préférence de 5 % à 50 % en poids, et tout particulièrement de 7 à 40 % en poids desdits copolymères séquencés à caractère élastique.

**22.** Composition selon l'une quelconque des revendications 11 à 21, **caractérisée par le fait que** ledit milieu physiologiquement acceptable comprend un ou plusieurs solvants appropriés choisis parmi l'eau, les cétones, les alcools, les alkylèneglycols, les éthers d'alkylèneglycol, les acétates d'alkyle en $C_{2-7}$, les éthers, les alcanes, les hydrocarbures aromatiques, les aldéhydes et les huiles volatiles.

**23.** Composition cosmétique selon l'une quelconque des revendications 11 à 22, **caractérisée par le fait que** ledit milieu physiologiquement acceptable comprend en outre une phase grasse composée de corps gras liquides ou solides à température ambiante, d'origine animale, végétale, minérale ou synthétique.

**24.** Composition cosmétique selon l'une quelconque des revendications 11 à 23, **caractérisée par le fait que** ledit milieu physiologiquement acceptable comprend en outre un ou plusieurs agents épaississants, un ou plusieurs polymères filmogènes et/ou un ou plusieurs agents plastifiants.

**25.** Composition cosmétique selon l'une quelconque des revendications 11 à 24, **caractérisée par le fait que** ledit milieu physiologiquement acceptable comprend en outre une phase particulaire constituée de pigments et/ou de nacres et/ou de charges.

**26.** Composition cosmétique selon l'une quelconque des revendications 11 à 25, **caractérisé par le fait que** ledit milieu physiologiquement acceptable comprend en outre un ou plusieurs additifs tels que des agents antioxydants, des parfums, des huiles essentielles, des conservateurs, des actifs cosmétiques lipophile ou hydrophiles, des agents hydratants, des vitamines, des colorants, des acides gras essentiels, des sphingolipides, des agents autobronzants, des filtres solaires, des agents anti-mousse, des agents séquestrants, ou des agents anti-radicalaires.

**27.** Composition cosmétique selon l'une quelconque des revendications 11 à 26, **caractérisée par le fait qu'**elle se présente sous forme de lotion, de suspension, de dispersion, de solution organique, aqueuse ou hydroalcoolique éventuellement épaissie ou gélifiée, de mousse, de spray, d'émulsion huile-dans-eau, eau-dans-huile ou multiple, de poudre libre, compacte ou coulée, de solide ou de pâte anhydre.

**28.** Composition cosmétique selon l'une quelconque des revendications 11 à 27, **caractérisée par le fait qu'**il s'agit d'une laque pour cheveux.

**29.** Composition cosmétique selon l'une quelconque des revendications 11 à 27, **caractérisée par le fait qu'**il s'agit d'un vernis à ongles.

**30.** Composition cosmétique selon l'une quelconque des revendications 11 à 27, **caractérisée par le fait qu'**il s'agit d'une composition de maquillage.

**31.** Utilisation selon l'une quelconque des revendications 1 à 10 pour améliorer le pouvoir coiffant et la souplesse d'une laque de cheveux.

**32.** Utilisation selon l'une quelconque des revendications 1 à 10 pour augmenter la résistance aux chocs d'un vernis à ongles.

**33.** Utilisation selon l'une quelconque des revendications 1 à 10, pour améliorer la tenue d'une composition de maquillage.

## Claims

**1.** Cosmetic use of block ethylenic copolymers of elastic nature, comprising

(a) at least one rigid block having a glass transition temperature ($T_g$) of greater than or equal to 20°C, consisting of units derived from one or more ethylenic monomers, and
(b) at least one flexible block having a glass transition temperature ($T_g$) of less than 20°C, consisting of units derived from one or more ethylenic monomers,

said copolymers allowing the production of a film having an instantaneous recovery of between 5% and 100% with the exclusion of block copolymers having flexible blocks consisting exclusively of ethylene, propylene, butylene, butadiene and/or isoprene units, chosen from diblock copolymers of formula AB, triblock copolymers of formula ABA or BAB and polyblock copolymers of formula $(AB)_n$, $B(AB)_n$ or $(AB)_nA$, in which each A represents a rigid block having a glass transition temperature of greater than or equal to room temperature (20°C), each B represents a flexible block having a glass transition temperature of less than room temperature (20°C) and n is at least equal to two, preferably equal to 2 or 3, the blocks A of the same polymer possibly being identical or different, and the blocks B of the same polymer possibly being identical or different.

**2.** Use according to Claim 1, **characterized in that** the block ethylenic copolymers of elastic nature are polymers obtained by controlled free-radical polymerization.

**3.** Use according to Claim 1 or 2, **characterized in that** said rigid block having a glass transition temperature ($T_g$) of greater than or equal to 20°C consists of units derived from one or more ethylenic monomers chosen from acrylic acid or methacrylic acid, $C_{1-20}$ alkyl methacrylates containing a linear, branched or cyclic chain, $C_{1-4}$ hydroxyalkyl methacrylates, certain vinyl esters, heterocyclic monomers, (meth)acrylamide, certain aliphatic, cycloaliphatic or aromatic methacrylamides, styrene, certain substituted styrenes, (meth)acrylic or vinyl monomers containing a fluoro or perfluoro group or (meth)acrylamides containing a fluoro or perfluoro group, (meth)acrylic or vinyl silicone monomers or silicone (meth)acrylamides, acrylic or vinyl monomers comprising an amine function that is optionally neutralized or quaternized, and ethylenic carboxybetaines or sulfobetaines.

**4.** Use according to one of the preceding claims, **characterized in that** said flexible block having a glass transition temperature ($T_g$) of less than 20°C consists of units derived from one or more ethylenic monomers chosen from $C_{1-20}$ alkyl acrylates containing a linear, branched or cyclic chain, $C_{6-20}$ aryl acrylates, $C_{1-4}$ hydroxyalkyl acrylates, mono-, di- or poly(ethylene glycol) (meth)acrylates containing an optionally etherified hydroxyl end, certain aliphatic,

cycloaliphatic or aromatic (meth)acrylamides, certain vinyl ethers, certain substituted styrenes, acrylic or vinyl monomers containing a fluoro or perfluoro group, and acrylic or vinyl silicone monomers.

**5.** Use according to any one of the preceding claims, **characterized in that** said ethylenic copolymers are triblock copolymers of formula ABA in which each A independently represents a rigid block having a glass transition temperature of greater than or equal to room temperature (20°C) and B represents a flexible block having a glass transition temperature which is less than room temperature (20°C).

**6.** Use according to any one of the preceding claims, **characterized in that** the block ethylenic copolymers are chosen from

- poly(methyl methacrylate-b-butyl acrylate-b-methyl methacrylate) triblock copolymers
- poly(methyl methacrylate-b-isobutyl acrylate-b-methyl methacrylate) triblock copolymers and
- poly(methyl methacrylate-b-butyl acrylate-b-styrene) triblock polymers.

**7.** Use according to any one of the preceding claims, **characterized in that** the rigid blocks A are incompatible, that is to say immiscible, with the flexible blocks B.

**8.** Use according to any one of the preceding claims, **characterized in that** the difference between the glass transition temperatures of the rigid blocks and the flexible blocks is at least equal to 20°C, preferably greater than 50°C and ideally greater than 100°C.

**9.** Use according to any one of the preceding claims, **characterized in that** said block polymers have an instantaneous recovery of between 5% and 95%, preferably between 10% and 90%, in particular between 20% and 80% and ideally between 55% and 78%.

**10.** Use according to any one of the preceding claims, **characterized in that** the blocks A represent from 10% to 60% by weight and in particular from 15% to 50% by weight of the final block copolymer and the blocks B represent from 40% to 90% by weight and in particular from 50% to 85% by weight of the final block copolymer.

**11.** Cosmetic composition comprising, in a physiologically acceptable medium, at least one block ethylenic copolymer of elastic nature comprising

(a) at least one rigid block having a glass transition temperature ($T_g$) of greater than or equal to 20°C, consisting of units derived from one or more ethylenic monomers, and
(b) at least one flexible block having a glass transition temperature ($T_g$) of less than 20°C, consisting of units derived from one or more ethylenic monomers,

said copolymers allowing the production of a film having an instantaneous recovery of between 5% and 100% with the exclusion of block copolymers having flexible blocks consisting exclusively of ethylene, propylene, butylene, butadiene and/or isoprene units, chosen from diblock copolymers of formula AB, triblock copolymers of formula ABA or BAB and polyblock copolymers of formula $(AB)_n$, in which each A represents a rigid block having a glass transition temperature of greater than or equal to room temperature (20°C), each B represents a flexible block having a glass transition temperature of less than room temperature (20°C) and n is at least equal to two, preferably equal to 2 or 3, the blocks A of the same polymer possibly being identical or different, and the blocks B of the same polymer possibly being identical or different.

**12.** Cosmetic composition according to Claim 11, **characterized in that** the block ethylenic copolymers of elastic nature are polymers obtained by controlled free-radical polymerization.

**13.** Composition according to Claim 11 or 12, **characterized in that** said rigid block having a glass transition temperature ($T_g$) of greater than or equal to 20°C consists of units derived from one or more ethylenic monomers chosen from acrylic acid or methacrylic acid, $C_{1-20}$ alkyl methacrylates containing a linear, branched or cyclic chain, $C_{1-4}$ hydroxyalkyl methacrylates, certain vinyl esters, heterocyclic monomers, (meth)acrylamide, certain aliphatic, cycloaliphatic or aromatic methacrylamides, styrene, certain substituted styrenes, (meth)acrylic or vinyl monomers containing a fluoro or perfluoro group or (meth)acrylamides containing a fluoro or perfluoro group, (meth)acrylic or vinyl silicone monomers or silicone (meth)acrylamides, acrylic or vinyl monomers comprising an amine function that is optionally neutralized or quaternized, and ethylenic carboxybetaines or sulfobetaines.

**14.** Cosmetic composition according to one of Claims 11 to 13, **characterized in that** said flexible block having a glass transition temperature ($T_g$) of less than 20°C consists of units derived from one or more ethylenic monomers chosen from $C_{1-20}$ alkyl acrylates containing a linear, branched or cyclic chain, $C_{6-20}$ aryl acrylates, $C_{1-4}$ hydroxyalkyl acrylates, mono-, di- or poly(ethylene glycol) (meth)acrylates containing an optionally etherified hydroxyl end, certain aliphatic, cycloaliphatic or aromatic (meth)acrylamides, certain vinyl ethers, certain substituted styrenes, acrylic or vinyl monomers containing a fluoro or perfluoro group, and acrylic or vinyl silicone monomers.

**15.** Compositions according to any one of Claims 11 to 14, **characterized in that** the ethylenic copolymers are triblock copolymers of formula ABA in which each A independently represents a rigid block having a glass transition temperature of greater than or equal to room temperature (20°C) and B represents a flexible block having a glass transition temperature which is less than room temperature (20°C).

**16.** Composition according to any one of Claims 11 to 15, **characterized in that** the rigid blocks A are incompatible, that is to say immiscible, with the flexible blocks B.

**17.** Cosmetic composition according to any one of Claims 11 to 16, **characterized in that** the ethylenic copolymers are chosen from

- poly(methyl methacrylate-b-butyl acrylate-b-methyl methacrylate) triblock copolymers
- poly(methyl methacrylate-b-isobutyl acrylate-b-methyl methacrylate) triblock copolymers and
- poly(methyl methacrylate-b-butyl acrylate-b-styrene) triblock polymers.

**18.** Composition according to any one of Claims 11 to 17, **characterized in that** the difference between the glass transition temperatures of the rigid blocks and the flexible blocks is at least equal to 20°C, preferably greater than 50°C and ideally greater than 100°C.

**19.** Composition according to any one of Claims 11 to 18, **characterized in that** said block polymers of elastic nature have an instantaneous recovery of between 5% and 95%, preferably between 10% and 90%, in particular between 20% and 80% and ideally between 55% and 78%.

**20.** Composition according to any one of Claims 11 to 19, **characterized in that** the blocks A represent from 10% to 60% by weight and in particular from 15% to 50% by weight of the final block copolymer and the blocks B represent from 40% to 90% by weight and in particular from 50% to 85% by weight of the final block copolymer.

**21.** Cosmetic composition according to any one of Claims 11 to 20, **characterized in that** it contains from 1% to 99% by weight, preferably from 5% to 50% by weight and most particularly from 7% to 40% by weight of said block copolymers of elastic nature.

**22.** Composition according to any one of Claims 11 to 21, **characterized in that** said physiologically acceptable medium comprises one or more suitable solvents chosen from water, ketones, alcohols, alkylene glycols, alkylene glycol ethers, $C_{2-7}$ alkyl acetates, ethers, alkanes, aromatic hydrocarbons, aldehydes and volatile oils.

**23.** Cosmetic composition according to any one of Claims 11 to 22, **characterized in that** said physiologically acceptable medium also comprises a fatty phase composed of fatty substances that are liquid or solid at room temperature, of animal, plant, mineral or synthetic origin.

**24.** Cosmetic composition according to any one of Claims 11 to 23, **characterized in that** said physiologically acceptable medium also comprises one or more thickeners, one or more film-forming polymers and/or one or more plasticizers.

**25.** Cosmetic composition according to any one of Claims 11 to 24, **characterized in that** said physiologically acceptable medium also comprises a particulate phase consisting of pigments and/or nacres and/or fillers.

**26.** Cosmetic composition according to any one of Claims 11 to 25, **characterized in that** said physiologically acceptable medium also comprises one or more additives such as antioxidants, fragrances, essential oils, preserving agents, lipophilic or hydrophilic cosmetic active agents, moisturizers, vitamins, dyes, essential fatty acids, sphingolipids, self-tanning agents, sunscreens, antifoams, sequestering agents or free-radical scavengers.

**27.** Cosmetic composition according to any one of Claims 11 to 26, **characterized in that** it is in the form of a lotion,

a suspension, a dispersion, an organic, aqueous or aqueous-alcoholic solution that is optionally thickened or gelled, a mousse, a spray, an oil-in-water, water-in-oil or multiple emulsion, a free, compact or cast powder, a solid or an anhydrous paste.

**28.** Cosmetic composition according to any one of Claims 11 to 27, **characterized in that** it is a hair lacquer.

**29.** Cosmetic composition according to any one of Claims 11 to 27, **characterized in that** it is a nail varnish.

**30.** Cosmetic composition according to any one of Claims 11 to 27, **characterized in that** it is a make-up composition.

**31.** Use according to any one of Claims 1 to 10, to improve the styling power and suppleness of a hair lacquer.

**32.** Use according to any one of Claims 1 to 10, to increase the impact strength of a nail varnish.

**33.** Use according to any one of Claims 1 to 10, to improve the hold of a make-up composition.

**Patentansprüche**

**1.** Verwendung von ethylenischen Sequenzcopolymeren mit elastischem Charakter, die die folgenden Sequenzen umfassen, in der Kosmetik:

(a) mindestens eine starre Sequenz mit einer Glasübergangstemperatur ($T_g$) von mindestens 20 °C, die aus Einheiten besteht, die von einem oder mehreren ethylenischen Monomeren abgeleitet sind, und
(b) mindestens eine weiche Sequenz, die eine Glasübergangstemperatur ($T_g$) unter 20 °C besitzt und aus Einheiten besteht, die von einem oder mehreren ethylenischen Monomeren abgeleitet sind,
wobei mit den Copolymeren ein Film mit einer sofortigen Bedeckung von 5 bis 100 % erhalten werden kann,
wobei sequentielle Copolymere ausgeschlossen sind, die weiche Sequenzen aufweisen, die ausschließlich aus den Einheiten Ethylen, Propylen, Butylen, Butadien und/oder Isopren bestehen,

die unter den disequentiellen Copolymeren der Formel AB, den trisequentiellen Copolymeren der Formel ABA oder BAB und den polysequentiellen Copolymeren der Formel $(AB)_n$, $B(AB)_n$ oder $(AB)_nA$ ausgewählt sind, wobei A jeweils eine starre Sequenz mit einer Glasübergangstemperatur von mindestens Raumtemperatur (20 °C), B jeweils eine weiche Sequenz mit einer Glasübergangstemperatur unter Raumtemperatur (20 °C) und n mindestens zwei und vorzugsweise 2 oder 3 bedeutet, wobei die Sequenzen A eines Polymers identisch oder voneinander verschieden sein können und die Sequenzen B eines Polymers identisch oder voneinander verschieden sein können.

**2.** Verwendung nach Anspruch 1, **dadurch gekennzeichnet, dass** die ethylenischen Sequenzcopolymere mit elastischem Charakter Polymere sind, die durch kontrollierte radikalische Polymerisation hergestellt werden.

**3.** Verwendung nach Anspruch 1 oder 2, **dadurch gekennzeichnet, dass** die starre Sequenz mit einer Glasübergangstemperatur ($T_g$) von mindestens 20 °C aus Einheiten besteht, die von einem oder mehreren ethylenischen Monomeren abgeleitet sind, die unter Acrylsäure, Methacrylsäure, $C_{1-20}$-Alkylmethacrylaten mit gerader, verzweigter oder cyclischer Kette, $C_{1-4}$-Hydroxyalkylmethacrylaten, einigen Vinylestern, heterocyclischen Monomeren, (Meth) acrylamiden, verschiedenen aliphatischen, cycloaliphatischen oder aromatischen Methacrylamiden, Styrol, verschiedenen substituierten Styrolen, (Meth)acrylmonomeren oder Vinylmonomeren mit fluorierter oder perfluorierter Gruppe oder (Meth)acrylamiden mit fluorierter oder perfluorierter Gruppe, siliconierten (Meth)acrylmonomeren oder Vinylmonomeren oder siliconierten (Meth)acrylamiden, Acrylmonomeren oder Vinylmonomeren, die eine gegebenenfalls neutralisierte oder quaternisierte Aminofunktion enthalten, und ethylenischen Carboxybetaine oder Sulfobetainen abgeleitet sind.

**4.** Verwendung nach einem der vorhergehenden Ansprüche, **dadurch gekennzeichnet, dass** die weiche Sequenz mit einer Glasübergangstemperatur ($T_g$) unter 20 °C aus Einheiten besteht, die von einem oder mehreren ethylenischen Monomeren abgeleitet sind, die unter den $C_{1-20}$-Alkylacrylaten mit gerader, verzweigter oder cyclischer Kette, $C_{6-20}$-Arylacrylaten, $C_{1-4}$-Hydroxyalkylacrylaten, Mono-, Di- oder Poly(ethylenglycol)(meth)acrylaten mit gegebenenfalls veretherter, endständiger Hydroxygruppe, verschiedenen aliphatischen, cycloaliphatischen oder aromatischen (Meth)acrylamiden, verschiedenen Vinylethern, verschiedenen substituierten Styrolen, Acrylmonomeren oder Vinylmonomeren mit fluorierter oder perfluorierter Gruppe und siliconierten Acrylmonomeren oder Vinylmonomeren ausgewählt sind.

**5.** Verwendung nach einem der vorhergehenden Ansprüche, **dadurch gekennzeichnet, dass** die ethylenischen Copolymere trisequentielle Copolymere der Formel ABA sind, worin die Sequenzen A jeweils unabhängig eine starre Sequenz mit einer Glasübergangstemperatur von mindestens Umgebungstemperatur (20 °C) bedeuten und B eine weiche Sequenz mit einer Glasübergangstemperatur unter Umgebungstemperatur (20 °C) ist.

**6.** Verwendung nach einem der vorhergehenden Ansprüche, **dadurch gekennzeichnet, dass** die ethylenischen Sequenzcopolymere unter den folgenden Polymeren ausgewählt sind:

- trisequentiellen Copolymeren Poly(methylmethacrylat-b-butylacrylat-b-methylmethacrylat),
- trisequentiellen Copolymeren Poly(methylmethacrylat-b-isobutylacrylat-b-methylmethacrylat), und
- trisequentiellen Polymeren Poly(methylmethacrylat-b-butylacrylat-b-styrol) .

**7.** Verwendung nach einem der vorhergehenden Ansprüche, **dadurch gekennzeichnet, dass** die starren Sequenzen A mit den weichen Sequenzen B inkompatibel, d. h. nicht mischbar sind.

**8.** Verwendung nach einem der vorhergehenden Ansprüche, **dadurch gekennzeichnet, dass** der Unterschied der Glasübergangstemperatur der starren Sequenzen und der weichen Sequenzen mindestens 20 °C beträgt und vorzugsweise über 50 °C und idealerweise über 100 °C liegt.

**9.** Verwendung nach einem der vorhergehenden Ansprüche, **dadurch gekennzeichnet, dass** die sequentiellen Polymere eine sofortige Bedeckung von 5 bis 95 %, vorzugsweise 10 bis 90 %, insbesondere 20 bis 80 % und idealerweise 55 bis 78 % aufweisen.

**10.** Verwendung nach einem der vorhergehenden Ansprüche, **dadurch gekennzeichnet, dass** die Sequenzen A 10 bis 60 Gew.-% und insbesondere 15 bis 50 Gew.-% des fertigen sequentiellen Copolymers und die Sequenzen B 40 bis 90 Gew.-% und insbesondere 50 bis 85 Gew.-% des fertigen sequentiellen Copolymers ausmachen.

**11.** Kosmetische Zusammensetzung, die in einem physiologisch akzeptablen Medium mindestens ein ethylenisches Sequenzcopolymer mit elastischem Charakter enthält, das aufweist

(a) mindestens eine starre Sequenz mit einer Glasübergangstemperatur ($T_g$) von mindestens 20 °C, die aus Einheiten besteht, die von einem oder mehreren ethylenischen Monomeren abgeleitet sind, und
(b) mindestens eine weiche Sequenz, die eine Glasübergangstemperatur ($T_g$) unter 20 °C besitzt und aus Einheiten besteht, die von einem oder mehreren ethylenischen Monomeren abgeleitet sind,
wobei mit den Copolymeren ein Film mit einer sofortigen Bedeckung von 5 bis 100 % erhalten werden kann, wobei Sequenzcopolymere ausgeschlossen sind, die weiche Sequenzen aufweisen, die ausschließlich aus den Einheiten Ethylen, Propylen, Butylen, Butadien und/oder Isopren bestehen,

die unter den disequentiellen Copolymeren der Formel AB, den trisequentiellen Copolymeren der Formel ABA oder BAB und den polysequentiellen Copolymeren der Formel $(AB)_n$, ausgewählt ist, wobei A jeweils eine starre Sequenz mit einer Glasübergangstemperatur von mindestens Raumtemperatur (20 °C), B jeweils eine weiche Sequenz mit einer Glasübergangstemperatur unter Raumtemperatur (20 °C) und n mindestens zwei und vorzugsweise 2 oder 3 bedeutet, wobei die Sequenzen A eines Polymers identisch oder voneinander verschieden sein können und die Sequenzen B eines Polymers identisch oder voneinander verschieden sein können.

**12.** Kosmetische Zusammensetzung nach Anspruch 11, **dadurch gekennzeichnet, dass** die ethylenischen Sequenzcopolymere mit elastischem Charakter durch kontrollierte radikalische Polymerisation hergestellte Polymere sind.

**13.** Zusammensetzung nach Anspruch 11 oder 12, **dadurch gekennzeichnet, dass** die starre Sequenz mit einer Glasübergangstemperatur ($T_g$) von mindestens 20 °C aus Einheiten besteht, die von einem oder mehreren ethylenischen Monomeren abgeleitet sind, die unter Acrylsäure, Methacrylsäure, $C_{1-20}$-Alkylmethacrylaten mit gerader, verzweigter oder cyclischer Kette, $C_{1-4}$-Hydroxyalkylmethacrylaten, einigen Vinylestern, heterocyclischen Monomeren, (Meth)acrylamiden, verschiedenen aliphatischen, cycloaliphatischen oder aromatischen Methacrylamiden, Styrol, verschiedenen substituierten Styrolen, (Meth)acrylmonomeren oder Vinylmonomeren mit fluorierter oder perfluorierter Gruppe oder (Meth)acrylamiden mit fluorierter oder perfluorierter Gruppe, siliconierten (Meth)acrylmonomeren oder Vinylmonomeren oder siliconierten (Meth)acrylamiden, Acrylmonomeren oder Vinylmonomeren,

die eine gegebenenfalls neutralisierte oder quaternisierte Aminofunktion enthalten, und ethylenischen Carboxybetaine oder Sulfobetainen abgeleitet sind.

**14.** Kosmetische Zusammensetzung nach einem der Ansprüche 11 bis 13, **dadurch gekennzeichnet, dass** die weiche Sequenz mit einer Glasübergangstemperatur ($T_g$) unter 20 °C aus Einheiten besteht, die von einem oder mehreren ethylenischen Monomeren abgeleitet sind, die unter den $C_{1-20}$-Alkylacrylaten mit gerader, verzweigter oder cyclischer Kette, $C_{6-20}$-Arylacrylaten, $C_{1-4}$-Hydroxyalkylacrylaten, Mono-, Di- oder Poly(ethylenglycol)(meth)-acrylaten mit gegebenenfalls veretherter, endständiger Hydroxygruppe, verschiedenen aliphatischen, cycloaliphatischen oder aromatischen (Meth)acrylamiden, verschiedenen Vinylethern, verschiedenen substituierten Styrolen, Acrylmonomeren oder Vinylmonomeren mit fluorierter oder perfluorierter Gruppe und siliconierten Acrylmonomeren oder Vinylmonomeren ausgewählt sind.

**15.** Zusammensetzung nach einem der Ansprüche 11 bis 14, **dadurch gekennzeichnet, dass** die ethylenischen Copolymere trisequentielle Copolymere der Formel ABA sind, worin die Sequenzen A jeweils unabhängig eine starre Sequenz mit einer Glasübergangstemperatur von mindestens Umgebungstemperatur (20 °C) bedeuten und B eine weiche Sequenz mit einer Glasübergangstemperatur unter Umgebungstemperatur (20 °C) ist.

**16.** Zusammensetzung nach einem der Ansprüche 11 bis 15, **dadurch gekennzeichnet, dass** die starren Sequenzen A mit den weichen Sequenzen B inkompatibel, d. h. nicht mischbar sind.

**17.** Kosmetische Zusammensetzung nach einem der Ansprüche 11 bis 16, **dadurch gekennzeichnet, dass** die ethylenischen Copolymere unter den folgenden Copolymeren ausgewählt sind

- trisequentiellen Copolymeren Poly(methylmethacrylat-b-butylacrylat-b-methylmethacrylat),
- trisequentiellen Copolymeren Poly(methylmethacrylat-b-isobutylacrylat-b-methylmethacrylat), und
- trisequentiellen Polymeren Poly(methylmethacrylat-b-butylacrylat-b-styrol).

**18.** Zusammensetzung nach einem der Ansprüche 11 bis 17, **dadurch gekennzeichnet, dass** der Unterschied der Glasübergangstemperatur der starren Sequenzen und der weichen Sequenzen mindestens 20 °C beträgt und vorzugsweise über 50 °C und idealerweise über 100 °C liegt.

**19.** Zusammensetzung nach einem der Ansprüche 11 bis 18, **dadurch gekennzeichnet, dass** die sequentiellen Polymere eine sofortige Bedeckung von 5 bis 95 %, vorzugsweise 10 bis 90 %, insbesondere 20 bis 80 % und idealerweise 55 bis 78 % aufweisen.

**20.** Zusammensetzung nach einem der Ansprüche 11 bis 19, **dadurch gekennzeichnet, dass** die Sequenzen A 10 bis 60 Gew.-% und insbesondere 15 bis 50 Gew.-% des fertigen Sequenzcopolymers und die Sequenzen B 40 bis 90 Gew.-% und insbesondere 50 bis 85 Gew.-% des fertigen Sequenzcopolymers ausmachen.

**21.** Kosmetische Zusammensetzung nach einem der Ansprüche 11 bis 20, **dadurch gekennzeichnet, dass** sie 1 bis 99 Gew.-%, vorzugsweise 5 bis 50 Gew.-% und insbesondere 7 bis 40 Gew.-% Sequenzcopolymere mit elastischem Charakter enthält.

**22.** Zusammensetzung nach einem der Ansprüche 11 bis 21, **dadurch gekennzeichnet, dass** das physiologisch akzeptable Medium ein oder mehrere geeignete Lösungsmittel enthält, die unter Wasser, Ketonen, Alkoholen, Alkylenglycolen, Alkylenglycolethern, $C_{2-7}$-Alkylacetaten, Ethern, Alkanen, aromatischen Kohlenwasserstoffen, Aldehyden und flüchtigen Ölen ausgewählt sind.

**23.** Kosmetische Zusammensetzung nach einem der Ansprüche 11 bis 22, **dadurch gekennzeichnet, dass** das physiologisch akzeptable Medium ferner eine Fettphase aufweist, die aus bei Umgebungstemperatur flüssigen oder festen Fettsubstanzen tierischer, pflanzlicher, mineralischer oder synthetischer Herkunft zusammengesetzt ist.

**24.** Kosmetische Zusammensetzung nach einem der Ansprüche 11 bis 23, **dadurch gekennzeichnet, dass** das physiologisch akzeptable Medium ferner ein oder mehrere Verdickungsmittel, ein oder mehrere filmbildende Polymere und/oder einen oder mehrere Weichmacher enthält.

**25.** Kosmetische Zusammensetzung nach einem der Ansprüche 11 bis 24, **dadurch gekennzeichnet, dass** das physiologisch akzeptable Medium ferner eine Partikelphase aufweist, die aus Pigmenten und/oder Perlglanzpigmenten

und/oder Füllstoffen besteht.

26. Kosmetische Zusammensetzung nach einem der Ansprüche 11 bis 25, **dadurch gekennzeichnet, dass** das physiologisch akzeptable Medium ferner einen oder mehrere Zusatzstoffe enthält, wie Antioxidantien, Parfums, etherische Öle, Konservierungsmittel, lipophile oder hydrophile kosmetische Wirkstoffe, Hydratisierungsmittel, Vitamine, Farbmittel, essentielle Fettsäuren, Sphingolipide, Selbstbräunungsmittel, Sonnenschutzfilter, Schaumverhütungsmittel, Maskierungsmittel oder Radikalfänger für freie Radikale.

27. Kosmetische Zusammensetzung nach einem der Ansprüche 11 bis 26, **dadurch gekennzeichnet, dass** sie als Lotion, Suspension, Dispersion, organische, wässrige oder wässrig-alkoholische, gegebenenfalls verdickte oder gelierte Lösung, Schaum, Spray, Öl-in-Wasser-Emulsion, Wasser-in-Öl-Emulsion, multiple Emulsion, loses, kompaktiertes oder gegossenes Pulver, Feststoff oder wasserfreie Paste vorliegt.

28. Kosmetische Zusammensetzung nach einem der Ansprüche 11 bis 27, **dadurch gekennzeichnet, dass** es sich um einen Haarlack handelt.

29. Kosmetische Zusammensetzung nach einem der Ansprüche 11 bis 27, **dadurch gekennzeichnet, dass** es sich um einen Nagellack handelt.

30. Kosmetische Zusammensetzung nach einem der Ansprüche 11 bis 27, **dadurch gekennzeichnet, dass** es sich um eine Zusammensetzung zum Schminken handelt.

31. Verwendung nach einem der Ansprüche 1 bis 10 zur Verbesserung des Frisiervermögens und der Geschmeidigkeit eines Haarlacks.

32. Verwendung nach einem der Ansprüche 1 bis 10 zur Erhöhung der Schlagfestigkeit eines Nagellacks.

33. Verwendung nach einem der Ansprüche 1 bis 10 zur Verbesserung der Haftung einer Zusammensetzung zum Schminken.